# Europäisches Patentamt
# European Patent Office
# Office européen des brevets

(11) Veröffentlichungsnummer: **0 240 797 B1**

(12) # EUROPÄISCHE PATENTSCHRIFT

(45) Veröffentlichungstag der Patentschrift:
**11.07.90**

(51) Int. Cl.⁵: **A61B 17/22**

(21) Anmeldenummer: **87104003.6**

(22) Anmeldetag: **18.03.87**

(54) Stosswellenquelle mit erhöhtem Wirkungsgrad.

(30) Priorität: **01.04.86 DE 3610837**

(43) Veröffentlichungstag der Anmeldung:
**14.10.87 Patentblatt 87/42**

(45) Bekanntmachung des Hinweises auf die Patenterteilung:
**11.07.90 Patentblatt 90/28**

(84) Benannte Vertragsstaaten:
**DE FR GB NL**

(56) Entgegenhaltungen:
EP-A- 183 236
EP-A- 0 130 709
EP-A- 0 133 665
FR-A- 2 325 266
GB-A- 534 448
JP-A-58 085 694

JOURNAL OF THE ACOUSTICAL SOCIETY OF AMERICA, Band 79, Nr. 2, Februar 1986, Seiten 566-570, Acoustical Society of America, Woodbury, New York, US; C.M. SEHGAL et al.: "Ultrasonic nonlinear parameters and sound speed of alcohol-water mixtures"

(73) Patentinhaber: **Siemens Aktiengesellschaft, Wittelsbacherplatz 2, D-8000 München 2(DE)**

(72) Erfinder: **Naser, Georg, Dipl.-Ing. (FH), Karlstrasse 10, D-8502 Zirndorf(DE)**
Erfinder: **Reichenberger, Helmut, Dr., Begonienstrasse 28, D-8501 Eckental(DE)**
Erfinder: **Schwark, Hubert, Heinrich-Hertz-Strasse 15, D-8520 Erlangen(DE)**

## Beschreibung

Die Erfindung betrifft eine Stoßwellenquelle, insbesondere für einen Lithotripter, mit einer Druckquelle zur Erzeugung eines Druckwellenimpulses, mit einer Fokussierungseinrichtung zur Fokussierung des Druckwellenimpulses und mit einer Abschlußmembran zum Einkoppeln des Druckwellenimpulses in einen Körper.

Eine solche Stoßwellenquelle ist aus der DE-OS 3 328 051 bekannt. Dort ist eine Druckquelle in Form eines Stoßwellenrohrs vorgesehen, welche einen Druckwellenimpuls abgibt. Der Druckwellenimpuls durchläuft eine Vorlaufstrecke bis zu einer Fokussierungseinrichtung, die z. B. als Linse ausgebildet ist.

Nach der Fokussierungseinrichtung durchläuft er eine Ankoppelstrecke, bevor er über eine Abschlußmembran in den Körper eines Patienten eingekoppelt wird. Hier dient er zur Lithotripsie, d.h. zur Zerstörung von Konkrementen, z.B. von Nierensteinen.

Eine Stoßwellenquelle der obengenannten Art ist weiterhin aus der deutschen Offenlegungsschrift 3 312 014 bekannt. Dort sind die Druckquelle und die Fokussierungseinrichtung zusammengefaßt. Eine konkav geformte Spule erzeugt einen bereits fokussierten Druckwellenimpuls. Die Fokussierungseinrichtung weist einen natürlichen oder geometrischen Fokus auf, an dessen Ort das zu zerstörende Konkrement plaziert wird. An diesem Fokus hat sich der Druckwellenimpuls zu einem Stoßwellenimpuls entwickelt.

Außerdem beschreibt die prioritätsältere, nicht vorveröffentlichte EP-A 0 183 236 eine Stoßwellenquelle, insbesondere für einen Lithotripter, mit einer Druckquelle zur Erzeugung eines Druckwellenimpulses, mit einer Fokussierungseinrichtung zur Fokussierung des Druckwellenimpulses und mit einer Abschlußmembran zum Einkoppeln des Druckwellenimpulses in einen Körper, bei der die Fokussierungseinrichtung durch einen Stoff gebildet ist, dessen B/A-Wert größer als der von Wasser ist und dessen akustische Impedanz kleiner als die von Wasser ist.

Hier durchgeführte Untersuchungen haben ergeben, daß allgemein folgendes gilt: Wenn bis zu einem Abstand von etwa einer halben Wellenlänge vom geometrischen Fokus entfernt noch keine Stoßwelle ausgebildet ist, dann kann aufgrund der Beugungsbegrenzung keine weitere Fokussierung und damit auch keine Ausbildung zur Stoßwelle bis hin zum Fokus mehr erfolgen. Die Wellenlänge ergibt sich dabei aus der Dauer der Grundschwingung, die dem momentanen Druckwellenimpuls zugeordnet ist. Untersuchungen haben weiter ergeben, daß der Druckwellenimpuls an der Fokussierungseinrichtung einen vorgegebenen Mindestwert des Verhältnisses von Amplitude zu Anstiegszeit haben muß, damit es überhaupt zur Ausbildung einer Stoßwelle kommt. Um diesen aufgrund der Geometrie und der üblichen Wasserfüllung der Verlaufstrecke vorgegebenen Mindestwert des Quotienten zu erreichen, ist es notwendig, den Druckwellenimpuls mit einer relativ hohen Amplitude am Ort der Fokussierungseinrichtung zu versehen. Dieses wirkt sich aus gerätetechnischer Sicht nachteilig auf die Lebensdauer der Druckquelle aus, z.B. einer elektromagnetischen Spule, und der Isolationsaufwand ist beträchtlich. Aus medizinischer Sicht ist die relativ hohe Belastung des Patienten mit akustischer Energie, also eine hohe Dosis, unerwünscht. Ein geringerer maximaler Druck im Zielgebiet wäre erstrebenswert.

Aufgabe vorliegender Erfindung ist es, eine Stoßwellenquelle der eingangs genannten Art so auszubilden, daß die genannten negativen Effekte reduziert werden.

Diese Aufgabe wird nach einer ersten Ausführungsform erfindungsgemäß dadurch gelöst, daß sich zwischen der Fokussierungseinrichtung und der Abschlußmembran ein Stoff befindet, dessen B/A-Wert größer oder gleich 9 ist und dessen akustische Impedanz kleiner oder gleich derjenigen von Wasser ist. Die stoffspezifische Größe B/A beschreibt für die Ausbreitung akustischer Wellen in einem flüssigen oder gasförmigen Medium die Abweichungen vom linearen Verhalten. Für Wasser hat B/A bei 20°C den Wert 5.

Gemäß einer zweiten Ausführungsform wird die Aufgabe erfindungsgemäß dadurch gelöst, daß die Fokussierungseinrichtung durch einen Stoff gebildet ist, dessen B/A-Wert größer oder gleich 9 ist und dessen akustische Impedanz kleiner als die von Wasser ist.

Für beide Ausführungsformen geeignete Substanzen sind Rizinusöl, Äthanol und Benzol mit B/A-Werten von 10, 10,5 bzw. 9.

Durch diese Maßnahme wird erreicht, daß sich der Druckwellenimpuls auf seinem Weg zur Abschlußmembran sehr viel schneller austeilt als bei einer üblichen Wasserfüllung. Es wird also in der Vorlaufstrecke schneller eine kürzere Anstiegszeit des Druckwellenimpulses erreicht. Dadurch ist es möglich, die Amplitude des Druckimpulses an der Druckquelle im Vergleich zu einem Lithotripter mit Wasserlaufstrecke zu senken.

An einem vorgegebenen Ort zwischen der Fokussierungseinrichtung und der Abschlußmembran kann bei geeigneter Einstellung trotz kleinerer Amplitude des von der Druckquelle erzeugten Druckwellenimpulses der gleiche Wert des Quotienten Amplitude zu Anstiegszeit des Druckwellenimpulses herrschen, als zuvor bei der Wasserfüllung.

Die kleinere, an der Druckquelle benötigte Amplitude des Druckwellenimpulses hat bei elektrischen Druckquellen eine geringere elektrische Spannung zur Erzeugung des Druckwellenimpulses zur Folge. Bei einer Druckquelle unter Einbau einer elektrischen Spule nach Art eines Stoßwellenrohres erhöht dies die Lebensdauer der Spule erheblich. Außerdem wird der Isolationsaufwand reduziert. Bei einer Funkenstrecke als Druckquelle wirkt sich eine niedrigere elektrische Spannung positiv auf den Abbrand der Funkenstrecke aus. Aufgrund der niedrigeren Ausgangsamplitude wird der Körper des Patienten mit einer kleineren Dosis akustischer Energie belastet. Dieses kann zu einer Reduzierung von möglichen Nebenerscheinungen einer Lithotripsiebehandlung, wie z. B. Reizung der Haut, an der Ankoppelstelle führen.

Weitere Vorteile und Ausgestaltungen der Erfindung ergeben sich aus der Beschreibung von Ausführungsbeispielen anhand der Figuren. Es zeigen:

Fig. 1 eine konkave Druckquelle mit Vorlaufstrecke und Ankoppelkörper und

Fig. 2 eine Druckquelle mit einer konvexen Abschlußmembran und Ankopplung über einen Gelkörper.

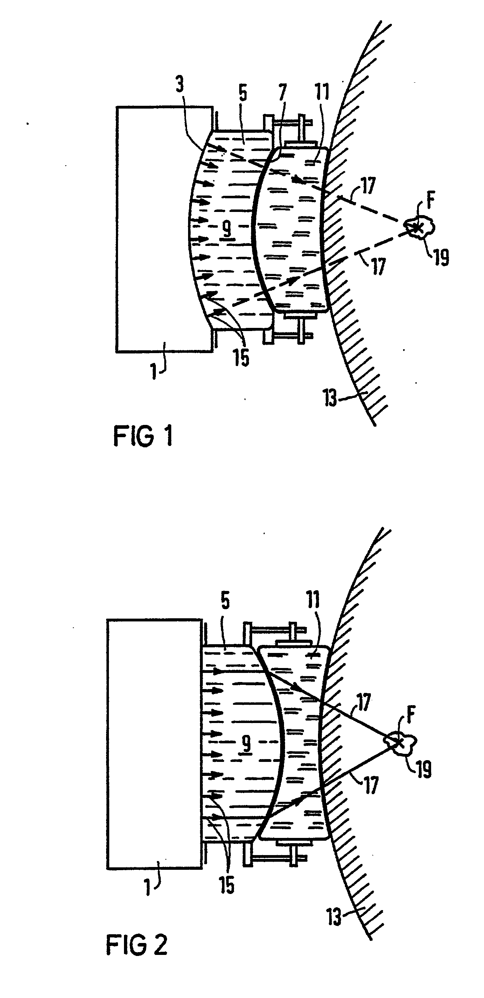

In Fig. 1 ist mit 1 eine Druckquelle bezeichnet, die gleichzeitig eine Fokussierungseinrichtung 3 beinhaltet oder als solche ausgebildet ist. Die Fokussierungseinrichtung 3 wird vorliegend durch die Formgebung der Druckquelle 1 realisiert. Insbesondere kann es sich dabei um eine (an sich bekannte) konkave Formgebung der Abstrahlfläche in einem Stoßwellenrohr handeln. Alternativ hierzu ist es möglich, die Druckquelle 1 und die Fokussierungseinrichtung 3 als getrennte Bauteile auszulegen. Die Druckquelle 1 wäre dann beispielsweise ein Stoßwellenrohr zur Erzeugung eines ebenen Druckwellenimpulses, und die Fokussierungseinrichtung 3 wäre eine akustische Linse oder ein Reflektor.

An die Druckquelle 1 mit integrierter Fokussierungseinrichtung 3 schließt sich eine Vorlaufstrecke 5 an, die von einer Abschlußmembran 7 begrenzt ist. Die Abschlußmembran 7 bildet zusammen mit der Druckquelle 1 einen Raum 9, welcher mit einem flüssigen Stoff gefüllt ist, der ein hohes B/A-Verhältnis und eine akustische Impedanz, die kleiner oder gleich der des Wassers ist, aufweist. An die Stirnseite der Abschlußmembran 7 grenzt ein geeigneter Ankoppelkörper 11 an, über welchen ein Druckwellenimpuls in den zu behandelnden Patienten 13 eingekoppelt wird. Die Anordnung von Druckquelle 1, Vorlaufstrecke 5 und Ankoppelkörper 11 weist einen geometrischen Fokus F auf.

Ein von der Druckquelle 1 mit integrierter Fokussierungseinrichtung 3 ausgesendeter Druckwellenimpuls 15 (durch kleine Pfeile angedeutet) nimmt einen Verlauf entlang der Linien 17. In dem Fokus F ist das zu zerstörende Konkrement 19 des Patienten 13 plaziert. Durch die Füllung des Raumes 9 mit dem Material mit hohem B/A-Verhältnis und mit einer akustischen Impedanz, die kleiner/gleich der des Wassers ist, wird eine schnelle Aufsteilung des Druckwellenimpulses 15 bei seinem Lauf durch den Raum 9 erreicht. Dadurch ist es möglich, die Amplitude des Druckwellenimpulses 15 an der Fokussierungseinrichtung 3 und damit auch an der Druckquelle 1 im Vergleich zur Verwendung von Wasser zu reduzieren und dennoch an einem vorgegebenen Punkt zwischen dem Fokus F und der Druckquelle 1 den Mindestwert des Quotienten aus Amplitude und Anstiegszeit des Druckwellenimpulses zu erreichen, um noch vor dem kritischen Abstand von etwa einer halben Wellenlänge die Ausbildung einer Stoßwelle zu erhalten. Der kritische Abstand kommt dadurch zustande, daß aufgrund der eintretenden Beugungsbegrenzung der Druckwellenimpuls nicht weiter fokussiert wird; damit kann es bis zum Fokus nicht mehr zur Ausbildung einer Stoßwelle kommen.

Durch die Reduzierung der Amplitude des Druckwellenimpulses 15 im Bereich der Fokussierungseinrichtung 3 und damit auch an der Druckquelle 1 ergibt sich aus gerätetechnischer Sicht eine Erhöhung der Lebensdauer der Druckquelle 1 und ein reduzierter elektrischer Isolationsaufwand, sofern es sich um eine elektrische Druckquelle 1 handelt. Gleichzeitig wird die Belastung des Patienten 13 mit akustischer Energie verringert. Der Wirkungsgrad der gesamten Stoßwellenquelle erhöht sich.

In Fig. 2 sind gleiche Bauteile mit denselben Bezugszeichen versehen wie in Fig. 1. Die Druckquelle 1 erzeugt hier einen ebenen Druckwellenimpuls 15. An die Druckquelle 1 schließt sich - zur Bildung einer Vorlaufstrecke 5 - eine Abschlußmembran 7 an, welche einen Raum 9 umschließt. Der Raum 9 ist vorzugsweise mit Äthanol gefüllt. Die stirnseitige Fläche der Abschlußmembran 7 ist konvex ausgebildet. An diese stirnseitig konvexe Fläche schließt sich ein entsprechend geformter Ankoppelkörper 11 aus einem geeigneten Material, z. B. aus einem Gel mit einer an den Körper des Patienten angepaßten akustischen Impedanz, an. Die freie Stirnseite des Ankoppelkörpers 11 ist an den Patienten 13 angelegt. Die gesamte Anordnung weist einen geometrischen Fokus F auf, in welchem wieder das Konkrement 19 plaziert ist. Der Druckwellenimpuls 15 nimmt einen Verlauf, der durch die Linien 17 angedeutet ist.

Das Äthanol im Raum 9 - anstelle von Wasser - beschleunigt die Aufsteilung des Druckwellenimpulses 15 erheblich. Dabei wird die zur Ausbildung einer Stoßwelle im Fokus notwendige Amplitude des anfänglichen Druckwellenimpulses 15 etwa auf die Hälfte reduziert.

Eine Äthanol-Vorlaufstrecke 5 in Verbindung mit einer (nicht gezeigten) flachkonkaven Druckquelle 1 und einer ebenen Trennfläche zum Ankoppelkörper 11 würde ähnlich fokussierend wirken wie die (in Fig. 2 gezeigte) ebene Druckquelle 1 mit konvexer Stirnseite an der Abschlußmembran 7.

Eine andere Verwendung der beschriebenen Stoßwellenquelle ist die Behandlung im Körper oder auf der Hand liegender Tumore mit Stoßwellen.

## Patentansprüche

1. Stoßwellenquelle, insbesondere für einen Lithotripter, mit einer Druckquelle zur Erzeugung eines Druckwellenimpulses, mit einer Fokussierungseinrichtung zur Fokussierung des Druckwellenimpulses, und mit einer Abschlußmembran zum Einkoppeln des Druckwellenimpulses in einen Körper, dadurch gekennzeichnet, daß sich zwischen der Fokussierungseinrichtung (3) und der Abschlußmembran (7) ein Stoff befindet, dessen B/A-Wert größer oder gleich 9 ist und dessen akustische Impedanz kleiner oder gleich derjenigen von Wasser ist.

2. Stoßwellenquelle nach Anspruch 1, dadurch gekennzeichnet, daß der B/A-Wert des zwischen der Fokussierungseinrichtung (3) und der Abschlußmembran (7) befindlichen Stoffes entweder 9 oder 10 oder 10,5 beträgt.

3. Stoßwellenquelle, insbesondere für einen Lithotripter, mit einer Druckquelle zur Erzeugung eines Druckwellenimpulses, mit einer Fokussierungseinrichtung zur Fokussierung des Druckwellenimpulses, und mit einer Abschlußmembran zum Einkoppeln des Druckwellenimpulses in einen Körper, dadurch gekennzeichnet, daß die Fokussierungseinrichtung durch einen Stoff gebildet ist, dessen B/A-Wert größer oder gleich 9 ist und dessen akustische Impedanz kleiner als die von Wasser ist.

4. Stoßwellenquelle nach Anspruch 3, dadurch gekennzeichnet, daß der B/A-Wert des die Fokussierungseinrichtung bildenden Stoffes entweder 9 oder 10 oder 10,5 beträgt.

5. Stoßwellenquelle nach Anspruch 1 oder 2, dadurch gekennzeichnet, daß der Stoff Rizinusöl, ein Alkohol wie Äthanol, oder Benzol ist.

## Claims

1. A shock wave source, more particularly for a lithotriptor, comprising a pressure source for generating a pressure wave pulse, a focusing device for focusing the pressure wave pulse and a seal diaphragm for coupling the pressure wave pulse into a body, characterised in that between the focusing device (3) and the seal diaphragm (7) is a substance, whose B/A value is 9 or above and whose acoustic impedance is less than or equal to that of water.

2. A shock wave source according to claim 1, characterised in that the B/A value of the substance arranged between the focusing device (3) and the seal diaphragm (7) is 9 or 10 or 10.5.

3. A shock wave source, more particularly for a lithotriptor, comprising a pressure source for generating a pressure wave pulse, a focusing device for focusing the pressure wave pulse and a seal diapghragm for coupling the pressure wave pulse into a body, characterised in that the focusing device is formed from a substance, whose B/A value is 9 or above and whose acoustic impedance is less than that of water.

4. A shock wave source according to claim 3, characterised in that the B/A value of the substance forming the focusing device is 9 or 10 or 10.5.

5. A shock wave source according to claim 1 or 2, characterised in that the substance is castor oil, an alcohol such as ethanol, or benzene.

## Revendications

1. Générateur d'ondes de choc, notamment pour un lithotriteur, comportant une source de pression servant à produire une impulsion d'onde de pression, un dispositif de focalisation servant à focaliser l'impulsion d'onde de pression et une membrane de fermeture pour l'injection par couplage de l'impulsion d'onde de pression dans un corps, caractérisé par le fait qu'entre le dispositif de focalisation (3) et la membrane de fermeture (16) est disposée une substance, dont la valeur B/A est supérieure ou égale à 9 et dont l'impédance acoustique est inférieure ou égale à celle de l'eau.

2. Générateur d'ondes de choc suivant la revendication 1, caractérisé par le fait que la valeur B/A de la substance située entre le dispositif de focalisation (3) et la membrane de fermeture (7) est égale à 9 ou à 10 ou à 10,5.

3. Générateur d'ondes de choc, notamment pour un lithotriteur, comportant une source de pression servant à produire une impulsion d'onde de pression, un dispositif de focalisation servant à focaliser l'impulsion d'onde de pression et une membrane de fermeture pour l'injection par couplage de l'impulsion d'onde de pression dans un corps, caractérisé par le fait que le dispositif de focalisation est formé par une substance, dont la valeur B/A est supérieure ou égale à 9 et dont l'impédance acoustique est inférieure à celle de l'eau.

4. Générateur d'ondes de choc selon la revendication 3, caractérisé par le fait que la valeur B/A de la substance constituant le dispositif de focalisation est égale à 9 ou à 10,5.

5. Générateur d'ondes de choc suivant la revendication 1 ou 2, caractérisé par le fait que la substance est de l'huile de ricin, un alcool tel que l'éthanol, ou du benzène.

FIG 1

FIG 2